# DEMANDE DE BREVET EUROPEEN

(11) **EP 1 738 746 A2**
(43) Date de publication de la demande: **03.01.2007**
(21) Numéro de dépôt: 06116421.6
(22) Date de dépôt: 30.06.2006
(51) Int. Cl.: A61K 8/49, A61Q 5/10

(54) **Utilisation de colorants azoïques comprenant une fonction sulfonamide ou amide pour la coloration de fibres kératiniques humaines et procede de coloration et composition tinctoriale les comprenant**

(30) Priorité: 30.06.2005 FR 0551843
(71) Demandeur: L'ORÉAL, 75008 Paris (FR)
(72) Inventeur: Lagrange, Alain, 77700 Coupvray (FR)
(74) Mandataire: Wattremez, Catherine

(57) **Abrégé**

La présente invention a pour objet l'utilisation pour la coloration de fibres kératiniques humaines d'une composition tinctoriale comprenant au moins un composé de formule (I) suivante : 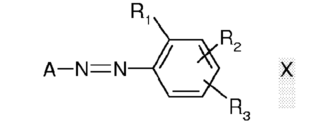 formule dans laquelle :
* **A** représente un hétérocycle aromatique monocationique éventuellement substitué comprenant 5 à 6 chaînons, comprenant au moins un atome d'azote et au moins un autre hétéroatome ; ledit hétérocycle étant éventuellement condensé avec un noyau aromatique non hétérocyclique ; ou représente un hétérocycle aromatique monocationique éventuellement substitué, à 6 chaînons, comprenant un atome d'azote, condensé avec un noyau aromatique non hétérocyclique à 6 chaînons ; le groupement A pouvant être rattaché à la fonction azoïque par l'intermédiaire de l'un des chaînons de l'hétérocycle ou du noyau aromatique non hétérocyclique ;
* **R₁,** représente -NH-CO-R'₁ ou -NH-SO₂-R'₂ ;
* **R₂** représente un atome d'hydrogène, un groupement alcoxy ; un groupement amino portant un ou deux substituants ; un groupement amino monosubstitué par un radical phényle éventuellement porteur d'un groupement alcoxy en C₁-C₄ linéaire ou ramifié, substitué par un groupement -D-NH-Φ(R₁)(R₃)-N=N-A ;
* **R₃** représente un atome d'hydrogène ; un radical alkyle ; un groupement alcoxy ; un groupement amino éventuellement substitué ;
*** lorsque R₂ et R₃** représentent deux groupements amino disubstitués portés par deux atomes de carbone adjacents, alors lesdits radicaux R₂ et R₃ peuvent former avec ces atomes de carbone, un hétérocycle éventuellement substitué ;
* au moins l'un des deux radicaux R₂ ou R₃ étant différent de l'hydrogène ;
* **X** représente un anion ou un mélange d'anions.

L'invention a de même pour objet un procédé de coloration de fibres kératiniques humaines dans lequel on met en contact une telle composition avec les fibres jusqu'à obtenir l'effet souhaité, en présence ou non d'un agent oxydant, ainsi qu'une composition tinctoriale comprenant au moins un composé de formule (I) et au moins un adjuvant.

## Description

La présente invention a pour objet l'utilisation de colorants mono- ou diazoïques comprenant au moins une fonction sulfonamide ou amide, pour la coloration de fibres kératiniques humaines ainsi qu'un procédé de coloration.

Il est connu de teindre les fibres kératiniques et en particulier les fibres kératiniques humaines, telles que les cheveux, avec des compositions tinctoriales contenant des colorants directs. Ces composés sont des molécules colorées et colorantes ayant une affinité pour les fibres. Il est connu par exemple d'utiliser des colorants directs du type nitrés benzéniques, des colorants anthraquinoniques, des nitropyridines, des colorants du type azoïque, xanthénique, acridinique, azinique ou triarylméthane.

Habituellement, ces colorants sont appliqués sur les fibres, éventuellement en présence d'un agent oxydant si l'on souhaite obtenir un effet simultané d'éclaircissement des fibres. Une fois le temps de pose écoulé, les fibres sont rincées, éventuellement lavées et séchées.

Les colorations qui résultent de l'utilisation de colorants directs sont des colorations temporaires ou semi-permanentes car la nature des interactions qui lient les colorants directs à la fibre kératinique, et leur désorption de la surface et/ou du coeur de la fibre sont responsables de leur faible puissance tinctoriale et de leur relative mauvaise tenue aux lavages ou à la transpiration.

Certains colorants directs peuvent de plus présenter des propriétés de photostabilité insuffisantes.

Le but de la présente invention est de fournir des colorants directs ne présentant pas les inconvénients des colorants directs existants.

La présente invention a donc pour objet l'utilisation pour la coloration de fibres kératiniques humaines d'une composition tinctoriale comprenant au moins un composé de formule (I) suivante, ou l'une de ses formes mésomères : 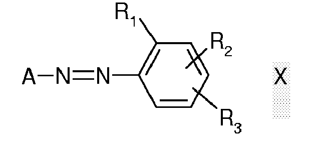 formule dans laquelle :
* **A** représente un hétérocycle aromatique monocationique éventuellement substitué comprenant 5 à 6 chaînons, comprenant au moins un atome d'azote et au moins un autre hétéroatome choisi parmi l'azote, le soufre ; ledit hétérocycle étant éventuellement condensé avec un noyau aromatique non hétérocyclique à 6 chaînons éventuellement substitué ; ou représente un hétérocycle aromatique monocationique éventuellement substitué, à 6 chaînons, comprenant un atome d'azote, condensé avec un noyau aromatique non hétérocyclique à 6 chaînons éventuellement substitué ; le groupement A pouvant être rattaché à la fonction azoïque par l'intermédiaire de l'un des chaînons de l'hétérocycle ou du noyau aromatique non hétérocyclique ;
* **R₁** représente :
   - -NH-CO-R'₁ avec R'₁ représentant :
      - un radical alkyle en C₁-C₆, linéaire ou ramifié, éventuellement porteur d'un groupement trialkyle (C₁-C₄) ammonium ou éventuellement porteur un groupement phénoxy ;
      - un groupement amino éventuellement porteur d'un ou plusieurs groupements identiques ou différents, choisi parmi les groupements alkyle en C₁-C₄, hydroxyalkyle en C₁-C₄, phényle ;
   - -NH-SO₂-R'₂ avec R'₂ représentant un radical alkyle en C₁-C₆, linéaire ou ramifié ;
* **R**₂ représente :
   - un atome d'hydrogène,
   - un groupement alcoxy en C₁-C₆ ;
   - un groupement amino portant un ou deux substituants identiques ou différents, choisis parmi :
      - les radicaux alkyle en C₁-C₆, linéaires ou ramifiés, éventuellement substitués par un groupement hydroxyle ; par un groupement alcoxy en C₁-C₄ ; par un groupement alcoxy(C₁-C₄)carbonyle ; par un groupement acyl(C₂-C₄)oxy ; par un groupement phényle ; par un groupement cyano ;
      - les deux radicaux alkyle dudit groupement amino pouvant former avec l'atome d'azote auquel ils sont rattachés, un hétérocycle à 5 ou 6 chaînons, saturé ou insaturé, comprenant éventuellement un autre hétéroatome identique ou différent de l'azote, comme par exemple l'oxygène ou encore un atome de soufre porteur de deux atomes d'oxygène et éventuellement substitué par un ou plusieurs radicaux alkyle en C₁-C₄ ;
      - les radicaux alkyle cycliques en C₅-C₇ ;
      - les radicaux naphtyle ;
      - un hétérocycle azoté à 5 ou 6 chaînons, éventuellement insaturé, rattaché au noyau aromatique par l'intermédiaire d'un atome d'azote, comprenant éventuellement un autre hétéroatome choisi parmi l'azote ou l'oxygène, ledit hétérocycle étant éventuellement substitué par un ou plusieurs radicaux alkyle en C₁-C₄ linéaires ou ramifiés ;
      - les radicaux phényle éventuellement substitué par un ou plusieurs groupements alcoxy en C₁-C₄, hydroxyalcoxy en C₁-C₄, cyano, alkyle en C₁-C₆ linéaire, ramifié ou cyclique, hydroxyle, amino, amino substitué par un ou deux radicaux alkyle en C₁-C₄ identiques ou non, alkyl(C₁-C₄)carbonyle, acétylamino, aminocarbonyle, carboxy, les atomes d'halogène ;
   - un groupement amino monosubstitué par un radical phényle éventuellement porteur d'un groupement alcoxy en C₁-C₄ linéaire ou ramifié, substitué par un groupement -D-NH-Φ(R₁)(R₃)-N=N-A avec D représentant un groupement -alkylène(C₁-C₂)-Φ- ou -NHCO-Φ- ou -O-alkylène (C₁-C₂)-O-Φ- ou -NH-CO-NH-Φ-ou une simple liaison ; R₁, et les hétérocycles A ayant la même définition que précédemment et choisis de préférence de telle sorte que les radicaux R₁ et les hétérocycles A soient respectivement identiques et placés dans les mêmes positions dans la molécule ; Φ représentant un radical phényle ; R₃ étant défini ci-dessous ;
* **R₃** représente :
   - un atome d'hydrogène ;
   - un radical alkyle en C₁-C₆ linéaire ou ramifié ;
   - un groupement alcoxy en C₁-C₆;
   - un groupement amino éventuellement porteur d'un ou plusieurs radicaux alkyle en C₁-C₄, hydroxyalkyle en C₁-C₄, phényle, identiques ou non ;
* **lorsque R₂ et R₃** représentent deux groupements amino disubstitués par deux radicaux alkyle identiques ou non, portés par deux atomes de carbone adjacents du noyau aromatique, alors lesdits radicaux R₂ et R₃ peuvent former avec les atomes de carbone auquel chacun est rattaché, un hétérocycle à 6 chaînons saturé éventuellement substitué par un ou deux radicaux alkyle en C₁-C₄, linéaires ou ramifiés, identiques ou non ; l'un des atomes de carbone dudit hétérocycle pouvant être remplacé par un groupement carbonyle ;
* au moins l'un des deux radicaux R₂ ou R₃ étant différent de l'hydrogène ;
* **X** représente un anion ou un mélange d'anions cosmétiquement acceptables.

La présente invention a de même pour objet un procédé de coloration de fibres kératiniques humaines consistant à mettre en contact une composition comprenant au moins un composé de formule (I) tel que défini précédemment, avec lesdites fibres sèches ou humides, pendant une durée suffisante pour obtenir l'effet souhaité.

Elle concerne enfin des compositions tinctoriales comprenant dans un milieu approprié pour la teinture des fibres kératiniques humaines au moins un colorant de formule (I) précitée et au moins un adjuvant choisi parmi les agents tensioactifs ; les polymères anioniques, cationiques, non ioniques, amphotères, zwittérioniques ou leurs mélanges ; les agents épaississants minéraux ; les polymères épaississants ; les agents antioxydants ; les agents de pénétration ; les agents séquestrants ; les parfums ; les tampons ; les agents dispersants ; les agents de conditionnement ; les agents filmogènes ; les céramides ; les agents conservateurs ; les agents opacifiants.

On a constaté que les composés de formule (I) tels que définis précédemment, présentent une bonne ténacité vis-à-vis d'agents extérieurs comme notamment les shampooings, ainsi qu'une faible sélectivité (on rappelle que la sélectivité représente la différence de montée de la couleur entre des zones d'un même cheveux ou entre des cheveux différemment sensibilisés).

Mais d'autres caractéristiques et avantages de la présente invention apparaîtront plus clairement à la lecture de la description et des exemples qui vont être présentés.

Dans ce qui va suivre et à moins qu'une autre indication ne soit donnée, les bornes délimitant un domaine de valeurs sont comprises dans ce domaine de valeurs.

Par ailleurs, les fibres kératiniques humaines font de préférence référence aux cheveux.

Dans la formule (I) précédemment définie, le groupement A représente plus particulièrement un hétérocycle choisi parmi les groupements imidazolium, pyrimidinium, pyrazolium, triazolium, thiazolium, thiadiazolium, quinolinium, benzimidazolium, benzoisothiazolium, benzothiazolium, éventuellement substitués.

De façon plus avantageuse, le groupement A représente un hétérocycle choisi parmi les groupements thiadiazolium, pyrazolium, thiazolium, benzimidazolium, benzoisothiazolium, benzothiazolium, triazolium, éventuellement substitués.

Conformément à un mode de réalisation plus particulier de l'invention, le groupement A représente l'un des hétérocycles suivants :

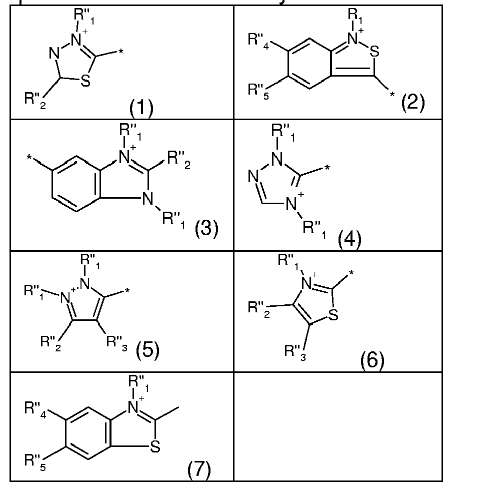

Dans lesquels
R"₁ représente :
- un radical alkyle en C₁-C₆ éventuellement substitué par un groupement hydroxyle, par un groupement cyano, par un groupement aminocarbonyle, par un groupement phényle ;
- un radical aryl(C₆)alkyle(C₁-C₆) ;
R"₂, R"₃, identiques ou différents, représentent :
- un atome d'hydrogène ;
- un groupement alkyle en C₁-C₆ linéaire ou ramifié ;
- un groupement phényle ;
- un groupement amino éventuellement porteur d'un ou deux radicaux identiques ou différents, choisis parmi :
   - les radicaux alkyle linéaires ou ramifiés en C₁-C₆ éventuellement substitués par un groupement hydroxyle, cyano ; ou cycliques en C₅-C₇ ;
   - les radicaux phényle ;
   - les groupements tétrahydro-1,1-dioxydo-3-thiényle ;
   - les deux radicaux alkyle du groupement amino pouvant former avec l'atome d'azote auquel ils sont rattachés, un hétérocycle de 5 à 7 chaînons comprenant éventuellement un autre hétéroatome différent ou non de l'azote ;
- un groupement alcoxy(C₁-C₆)carbonyle ;
R"₄, R"₅, identiques ou différents, représentent :
- un atome d'hydrogène ;
- un groupement alkyle en C₁-C₆ linéaire ou ramifié ;
- un groupement alcoxy en C₁-C₆ linéaire ou ramifié ;
- un atome d'halogène, de préférence le chlore ;
- un groupement nitro.

De préférence, le groupement A est choisi parmi les groupements thiadiazolium, pyrazolium, thiazolium, benzimidazolium, triazolium, éventuellement substitués.

Conformément à un mode de réalisation particulier de l'invention, le radical R₂ est en position para par rapport à la fonction azoïque.

Selon un autre mode de réalisation, le radical R₃ est en position méta par rapport à la fonction azoïque.

De préférence, R₃ représente un atome d'hydrogène ou un radical alcoxy, avantageusement méthoxy.

Selon un autre mode de réalisation préféré, R₂ désigne un radical amino substitué.

De préférence, R₁ représente un radical acétylamino ou méthanesulfonamide (ou mésylamino).

Plus particulièrement, l'anion ou le mélange d'anions cosmétiquement acceptables, X, est (sont) choisi(s) parmi les halogénures tels que chlorures, bromures, fluorures, iodures ; les hydroxyde ; les sulfates ; les hydrogénosulfates ; les alkyl(C₁-C₆)sulfates ; les phosphates ; les carbonates ; les hydrogénocarbonates ; les perchlorates ; les acétates ; les tartrates ; les citrates, les oxalates ; les alkyl(C₁-C₆)sulfonates tels que le méthylsulfonate ; les arylsulfonates substitués ou non par un radical alkyle en C₁-C₄ tels que par exemple un 4-toluylsulfonate.

De préférence, le composé de formule (I) est choisi parmi les composés suivants :

| | |
|---|---|
| 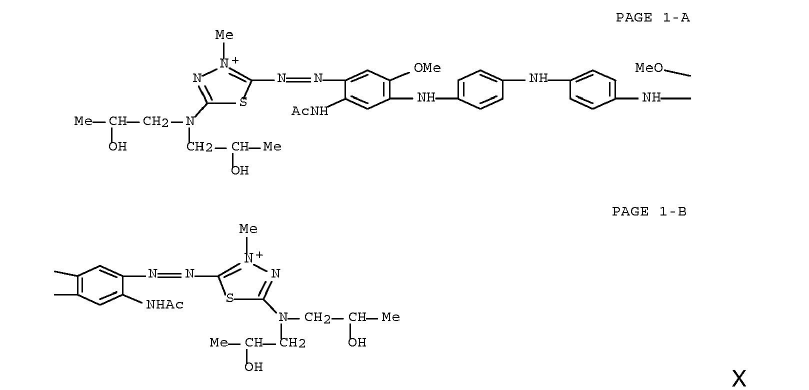 | |
| Sel de 1,3,4-Thiadiazolium, 2,2'- [iminobis[4,1-phenylene imino[2-(acetylamino)-5-methoxy-4,1-phenylene]azo]]bis [5-[bis(2-hydroxypropyl)amino]-3-methyle | |
| 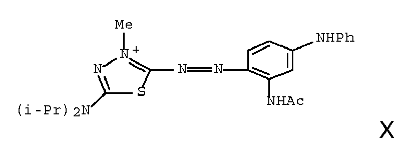 | 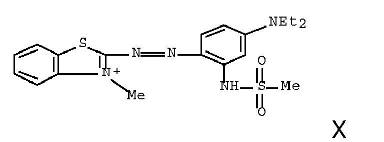 |
| Sel de 1,3,4-Thiadiazolium, 2-[[2-(acetylamino)-4-(phenyl amino)phenyl]azo]-5-[bis(1-methylethyl)amino]-3-methyle | Sel de Benzothiazolium, 2-[[4-(diethylamino)-2-[(methylsulfonyl)amino]phenyl]azo]- 3-methyle |
| 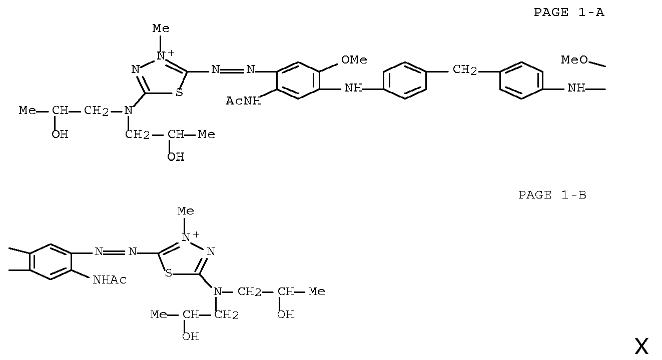 | |
| Sel de 1,3,4-Thiadiazolium, 2,2'-[methylenebis[4,1-phenyleneimino[2-(acetylamino)- 5-methoxy-4,1-phenylene]azo]]bis[5-[bis(2-hydroxypropyl)amino]-3-methyle | |
| 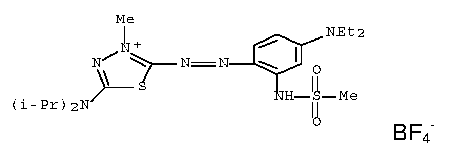 | 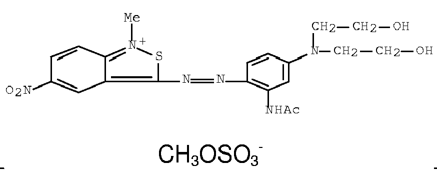 |
| Tétrafluoroborate de 1,3,4-Thiadiazolium, 5-[bis(1-methylethyl)amino]-2-[[4-(diethylamino)-2-[(methylsulfonyl)amino]phenyl]azo]-3-methyle | Méthylsulfate de 2,1-Benzisothiazolium, 3-[[2-(acetylamino)-4-[bis(2-hydroxyethyl)amino]phenyl]azo]-1-methyl-5-nitro |
| 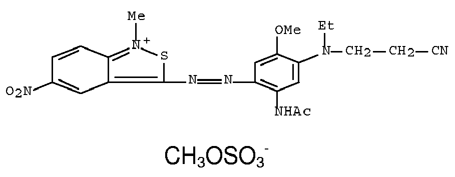 | 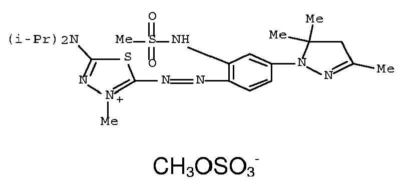 |
| Méthylsulfate de 2,1-Benzisothiazolium, 3-[[2-(acetylamino)-4-[(2-cyanoethyl)ethylamino]-5-methoxyphenyl]azo]-1-methyl-5-nitro | Méthylsulfate de 1,3,4-Thiadiazolium, 5-[bis(1-methylethyl)amino]-2-[[4-(4,5-dihydro-3,5,5-trimethyl-1H-pyrazol-1-yl)-2-[(methylsulfonyl)amino] phenyl]azo]-3-methyle |
| 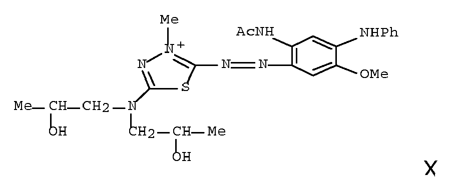 | 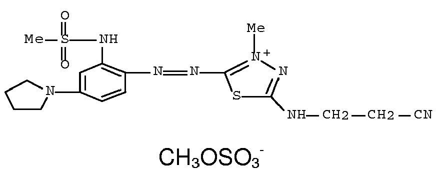 |
| Sel de 1,3,4-Thiadiazolium, 2-[[2-(acetylamino)-5-methoxy-4-(phenylamino)phenyl]azo]-5-[bis(2-hydroxypropyl)amino]-3-methyle | Méthylsulfate de 1,3,4-Thiadiazolium, 5-[(2-cyanoethyl)amino]-3-methyl-2-[[2-[(methylsulfonyl)amino]-4-(1-pyrrolidinyl)phenyl]azo] |
| 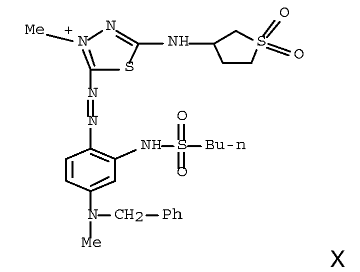 | 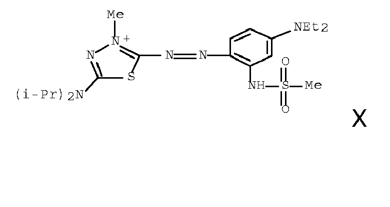 |
| Sel de 1,3,4-Thiadiazolium, 2-[[2-[(butylsulfonyl)amino]-4-[methyl(phenylmethyl)amino]phenyl]azo]-3-methyl-5-[(tetrahydro-1,1-dioxido-3-thienyl)amino] | Sel de 1,3,4-Thiadiazolium, 5-[bis(1-methylethyl)amino]-2-[[4-(diethylamino)-2-[(methylsulfonyl)amino]phenyl]azo]-3-methyle |
| 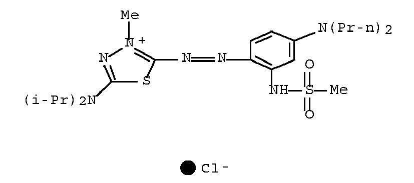 | 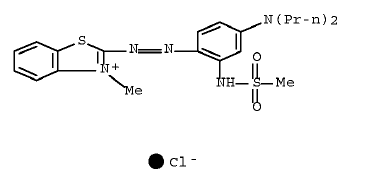 |
| Chlorure de 1,3,4-Thiadiazolium, 5-[bis(1-methylethyl) amino]-2-[[4-(dipropylamino)-2-[(methylsulfonyl)amino] phenyl]azo]-3-methyle | Chlorure de Benzothiazolium, 2-[[4-(dipropylamino)-2-[(methylsulfonyl)amino]phenyl]azo]-3-methyle |
| 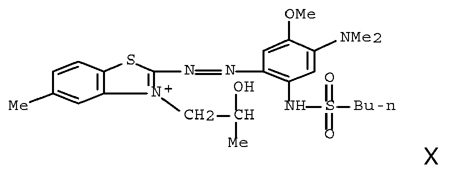 | 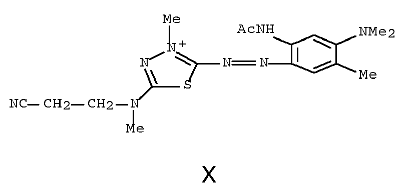 |
| Sel de Benzothiazolium, 2-[[2-[(butylsulfonyl)amino]-4-(dimethylamino)-5-methoxyphenyl]azo]-3-(2-hydroxypropyl)-5-methyle | Sel de 1,3,4-Thiadiazolium, 2-[[2-(acetylamino)-4-(dimethylamino)-5-methylphenyl]azo]-5-[(2-cyanoethyl)methylamino]-3-methyle |
| 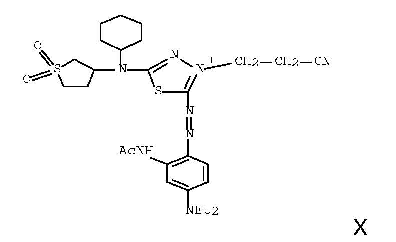 | 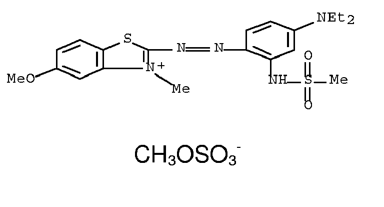 |
| Sel de 1,3,4-Thiadiazolium, 2-[[2-(acetylamino)-4-(diethylamino)phenyl]azo]-3-(2-cyanoethyl)-5-[cyclohexyl(tetrahydro-1,1-dioxido-3-thienyl)amino] | Méthylsulfate de Benzothiazolium, 2-[[4-(diethylamino)-2-[(methylsulfonyl)amino]phenyl]azo]- 5-methoxy-3-methyle |
| 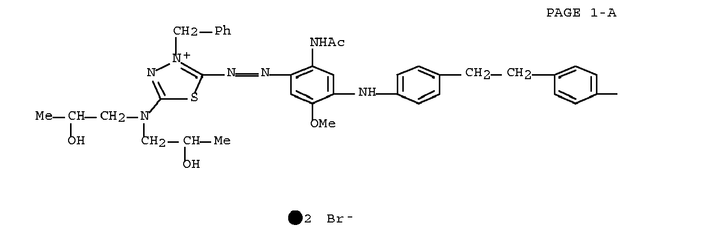 | |
| 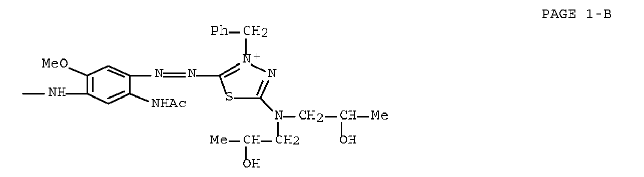 | |
| Dibromure de 1,3,4-Thiadiazolium, 2,2'-[1,2-ethanediylbis[4,1-phenyleneimino[2-(acetylamino)-5-methoxy-4,1-phenylene]azo]]bis[5-[bis(2-hydroxypropyl)amino]-3-(phenylmethyle) | |
| 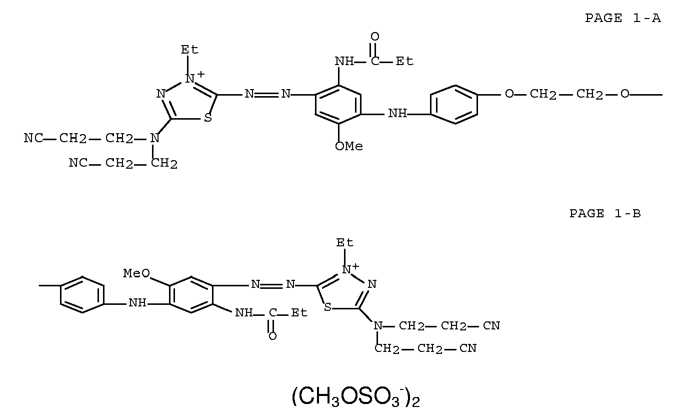 | |
| Bis(méthylsulfate) de 1,3,4-Thiadiazolium, 2,2'-[1,2-ethanediylbis[oxy-4,1-phenyleneimino[5-methoxy-2-[(1-oxopropyl)amino]-4,1-phenylene]azo]]bis[5-[bis(2-cyanoethyl)amino]-3-ethyle | |
| 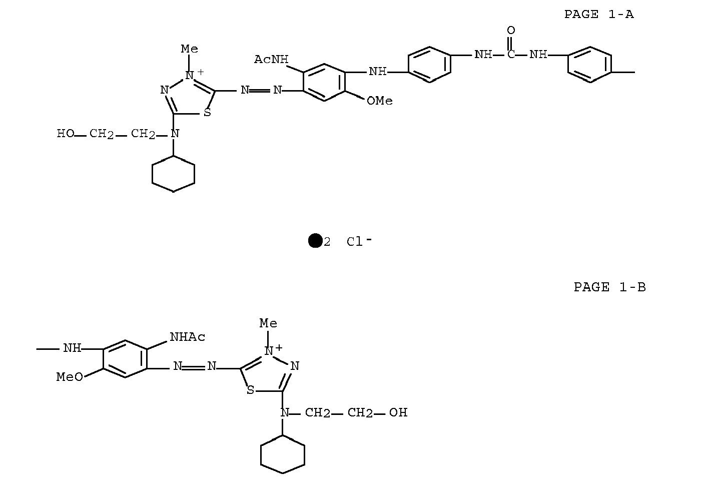 | |
| Dichlorure de 1,3,4-Thiadiazolium, 2,2'-[carbonylbis[imino-4,1-phenyleneimino[2-(acetylamino)-5-methoxy-4,1-phenylene]azo]]bis[5-[cyclohexyl(2-hydroxyethyl)amino]-3-methyle | |
| 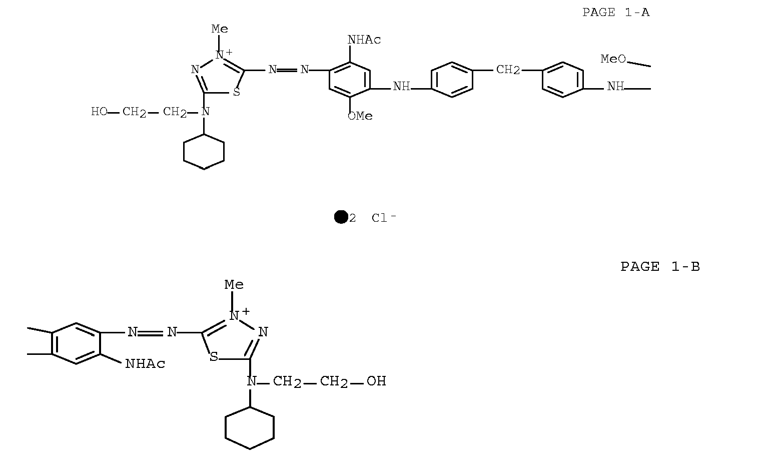 | |
| Dichlorure de 1,3,4-Thiadiazolium, 2,2'-[methylenebis[4,1-phenyleneimino[2-(acetylamino)- 5-methoxy-4,1-phenylene]azo]]bis[5-[cyclohexyl(2-hydroxyethyl)amino]-3-methyle | |
| 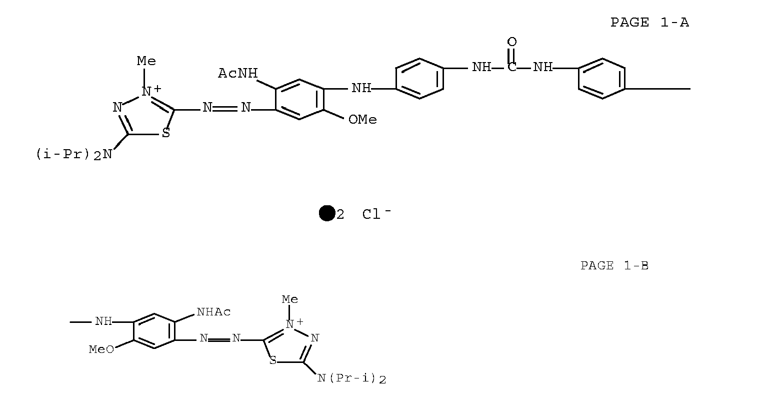 | |
| Dichlorure de 1,3,4-Thiadiazolium, 2,2'-[carbonylbis[imino-4,1-phenyleneimino[2-(acetylamino)-5-methoxy-4,1-phenylene]azo]]bis[5-[bis(1-methylethyl)amino]-3-methyle | |
| 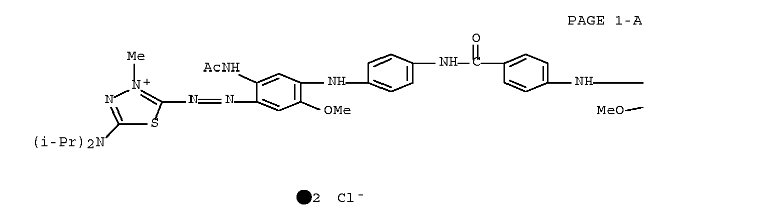 | |
| 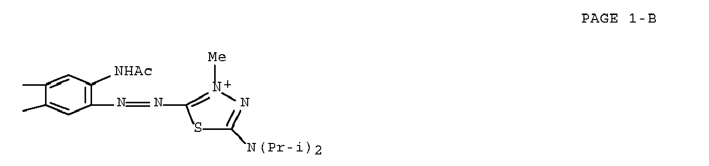 | |
| Dichlorure de 1,3,4-Thiadiazolium, 2-[[2-(acetyl amino)-4-[[4-[[[4-[[5-(acetylamino)-4-[[5-[bis(1-methylethyl)amino]-3-methyl-1,3,4-thiadiazolium-2-yl]azo]-2-methoxyphenyl]amino]phenyl]amino] carbonyl]phenyl]amino]-5-methoxyphenyl]azo]-5-[bis(1-methylethyl)amino]-3-methyle | |
| 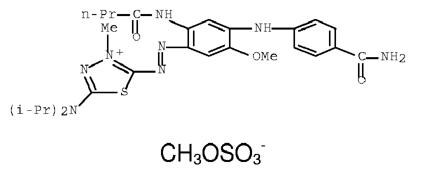 | 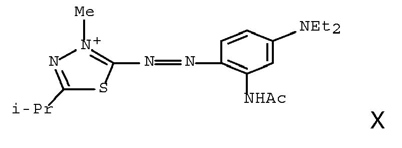 |
| Méthylsulfate de 1,3,4-Thiadiazolium, 2-[[4-[[4-(aminocarbonyl)phenyl]amino]-5-methoxy-2- [(1-oxobutyl)amino]phenyl]azo]-5-[bis(1-methylethyl)amino]-3-methyle | Sel de 1,3,4-Thiadiazolium, 2-[[2-(acetylamino)-4-(diethylamino)phenyl]azo]-3-methyl-5-(1-methylethyle) |
| 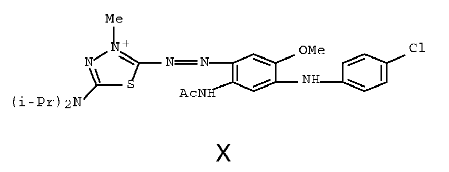 | 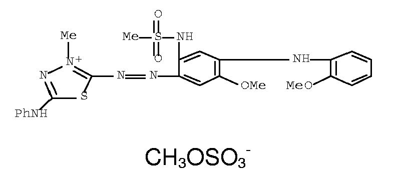 |
| Sel de 1,3,4-Thiadiazolium, 2-[[2-(acetylamino)-4-[(4-chlorophenyl)amino]-5-methoxyphenyl]azo]-5-[bis(1-methylethyl)amino]-3-methyle | Méthylsulfate de 1,3,4-Thiadiazolium, 2-[[5-methoxy-4-[(2-methoxyphenyl)amino]-2- [(methylsulfonyl)amino]phenyl]azo]-3-methyl-5-(phenylamino) |
| 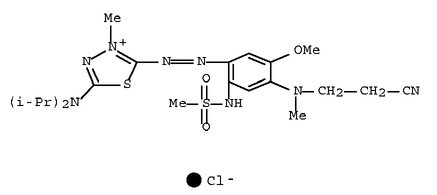 | 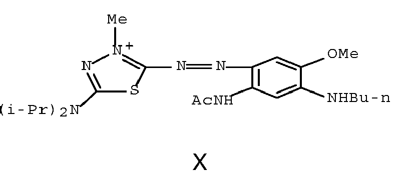 |
| Chlorure de 1,3,4-Thiadiazolium, 5-[bis(1-methylethyl)amino]-2-[[4-[(2-cyanoethyl)methylamino]-5-methoxy-2-[(methylsulfonyl)amino]phenyl]azo]-3-methyle | Sel de 1,3,4-Thiadiazolium, 2-[[2-(acetylamino)-4-(butylamino)-5methoxyphenyl]azo]-5-[bis(1-methylethyl)amino]-3-methyle |
| 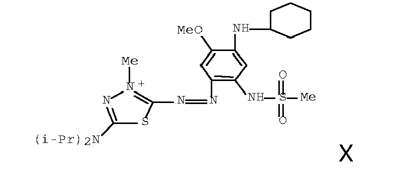 | 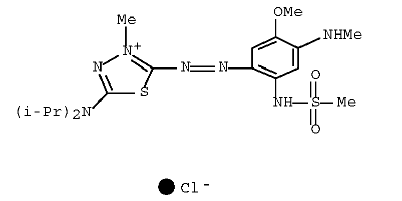 |
| Sel de 1,3,4-Thiadiazolium, 5-[bis(1-methylethyl)amino]-2-[[4-(cyclohexylamino)-5-methoxy-2-[(methylsulfonyl)amino]phenyl]azo]-3-methyle | Chlorure de 1,3,4-Thiadiazolium, 5-[bis(1-methylethyl)amino]-2-[[5-methoxy-4-(methylamino)-2-[(methylsulfonyl)amino]phenyl]azo]-3-methyle |
| 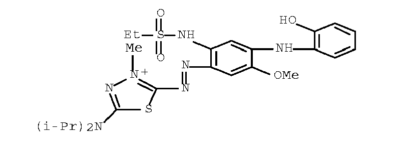 | 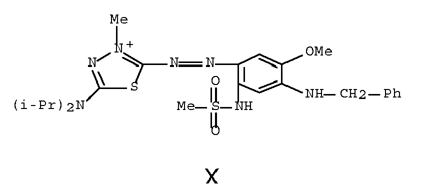 |
| Sel de 1,3,4-Thiadiazolium, 5-[bis(1-methylethyl)amino]-2-[[2-[(ethylsulfonyl)amino]-4-[(2-hydroxyphenyl)amino]-5-methoxyphenyl]azo]-3-methyle | Sel de 1,3,4-Thiadiazolium, 5-[bis(1-methy lethyl)amino]-2-[[5-methoxy-2-[(methylsulfonyl) amino]-4-[(phenylmethyl)amino]phenyl]azo]-3-methyle |
| 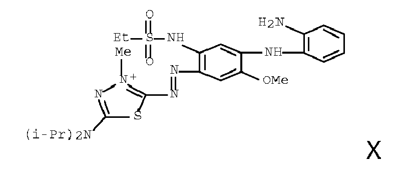 | 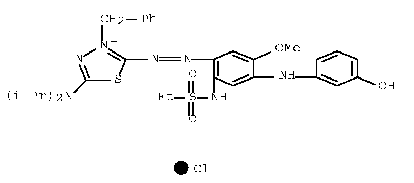 |
| Sel de 1,3,4-Thiadiazolium, 2-[[4-[(2-aminophenyl)amino]-2-[(ethylsulfonyl)amino]- 5-methoxyphenyl]azo]-5-[bis(1-methylethyl)amino]-3-methyle | Chlorure de 1,3,4-Thiadiazolium, 5-[bis(1-methylethyl)amino]-2-[[2-[(ethylsulfonyl)amino]-4-[(3-hydroxyphenyl)amino]-5-methoxyphenyl]azo]-3- (phenylmethyle) |
| 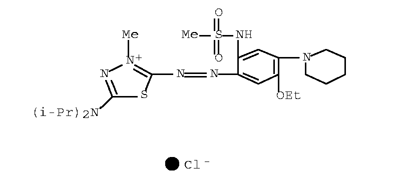 | 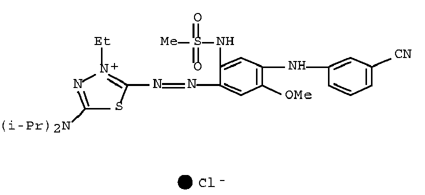 |
| Chlorure de 1,3,4-Thiadiazolium, 5-[bis(1-methylethyl)amino]-2-[[5-ethoxy-2-[(methylsulfonyl)amino]-4-(1-piperidinyl)phenyl]azo]-3-methyle | Chlorure de 1,3,4-Thiadiazolium, 5-[bis(1-methylethyl)amino]-2-[[4-[(3-cyanophenyl)amino]-5-methoxy-2-[(methylsulfonyl)amino]phenyl]azo]-3-ethyle |
| 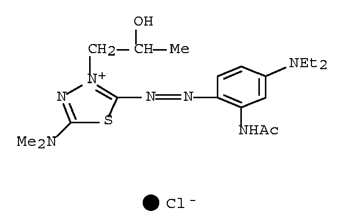 | 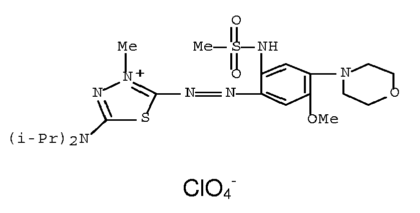 |
| Chlorure de 1,3,4-Thiadiazolium, 2-[[2-(acetylamino)-4-(diethylamino)phenyl]azo]-5- (dimethylamino)-3-(2-hydroxypropyle) | Perchlorate de 1,3,4-Thiadiazolium, 5-[bis(1-methylethyl)amino]-2-[[5-methoxy-2-[(methyl sulfonyl)amino]-4-(4-morpholinyl)phenyl]azo]-3-methyle |
| 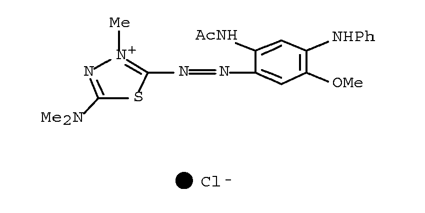 | 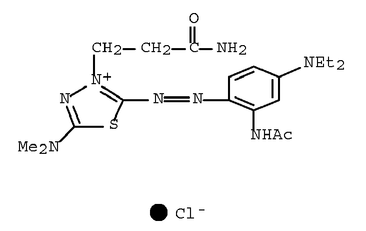 |
| Chlorure de 1,3,4-Thiadiazolium, 2-[[2-(acetylamino)-5-methoxy-4-(phenylamino)phenyl]azo]-5-(dimethylamino)-3-methyle | Chlorure de 1,3,4-Thiadiazolium, 2-[[2-(acetylamino)-4-(diethylamino)phenyl]azo]-3-(3-amino-3-oxopropyl)-5-(dimethylamino) |
| 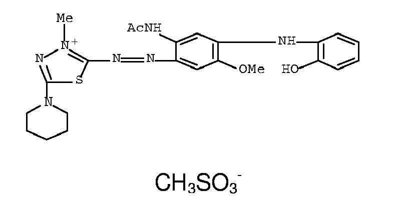 | 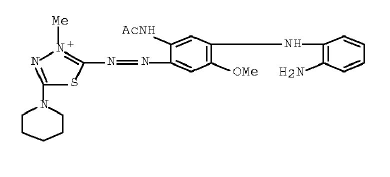 |
| Méthylsulfate de 1,3,4-Thiadiazolium, 2-[[2-(acetylamino)-4-[(2-hydroxyphenyl)amino]-5-methoxyphenyl]azo]-3-methyl-5-(1-piperidinyle) | Sel de 1,3,4-Thiadiazolium, 2-[[2-(acetylamino)-4-[(2-aminophenyl)amino]-5-methoxyphenyl]azo]-3-methyl-5-(1-piperidinyle |
| 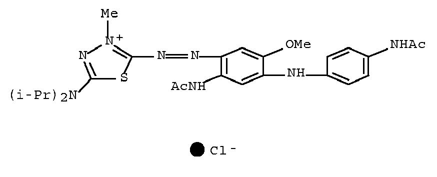 | 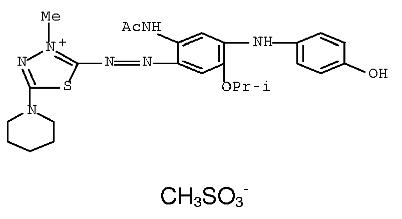 |
| Chlorure de 1,3,4-Thiadiazolium, 2-[[2-(acetylamino)-4-[[4-(acetylamino)phenyl]amino]- 5-methoxyphenyl]azo]-5-[bis(1-methylethyl)amino]-3-methyle | Méthylsulfate de 1,3,4-Thiadiazolium, 2-[[2-(acetylamino)-4-[(4-hydroxyphenyl)amino]-5-(1-methylethoxy)phenyl]azo]-3-methyl-5-(1-piperidinyle) |
| 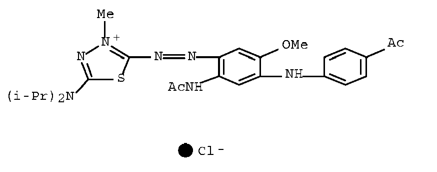 | 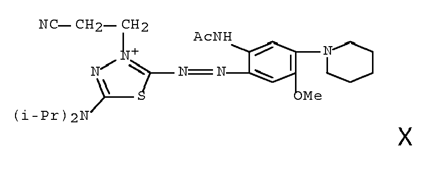 |
| Chlorure de 1,3,4-Thiadiazolium, 2-[[2-(acetylamino)-4-[(4-acetylphenyl)amino]-5-methoxyphenyl]azo]-5-[bis(1-methylethyl)amino]-3-methyle | Sel de 1,3,4-Thiadiazolium, 2-[[2-(acetylamino)-5-methoxy-4-(1-piperidinyl)phenyl]azo]-5-[bis(1-methylethyl)amino]-3-(2-cyanoethyle) |
| 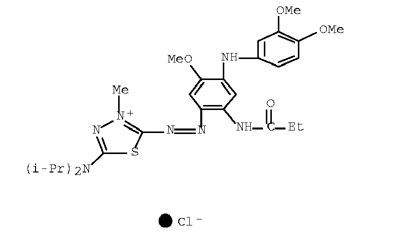 | 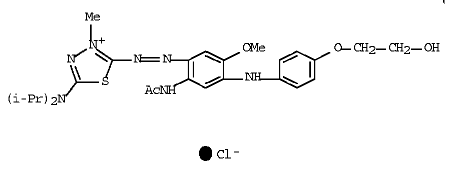 |
| Chlorure de 1,3,4-Thiadiazolium, 5-[bis(1-methylethyl)amino]-2-[[4-[(3,4-dimethoxyphenyl)amino]-5-methoxy-2-[(1-oxopropyl)amino]phenyl]azo]-3-methyle | Chlorure de 1,3,4-Thiadiazolium, 2-[[2-(acetylamino)-4-[[4-(2-hydroxyethoxy)phenyl]amino]-5-methoxy phenyl]azo]-5-[bis(1-methylethyl)amino]-3-methyle |
| 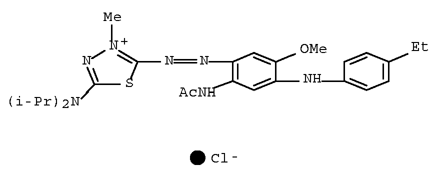 | 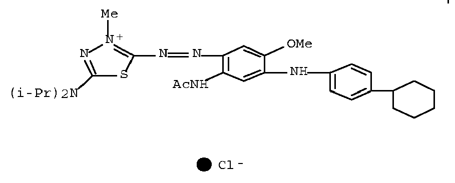 |
| Chlorure de 1,3,4-Thiadiazolium, 2-[[2-(acetylamino)-4-[(4-ethylphenyl)amino]-5-methoxyphenyl]azo]-5-[bis(1-methylethyl)amino]-3-methyle | Chlorure de 1,3,4-Thiadiazolium, 2-[[2-(acetylamino)-4-[(4-cyclohexylphenyl)amino]-5-methoxyphenyl]azo]-5-[bis(1-methylethyl)amino]-3-methyle |
| 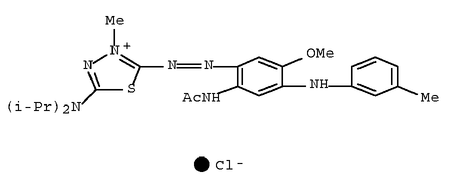 | 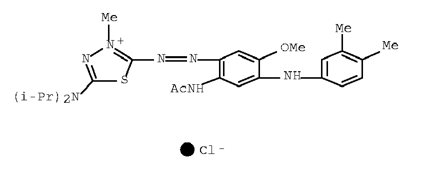 |
| Chlorure de 1,3,4-Thiadiazolium, 2-[[2-(acetylamino)-5-methoxy-4-[(3-methylphenyl)amino]phenyl]azo]-5-[bis(1-methylethyl)amino]-3-methyle | Chlorure de 1,3,4-Thiadiazolium, 2-[[2-(acetylamino)-4-[(3,4-dimethylphenyl)amino]-5-methoxyphenyl] azo]-5-[bis(1-methylethyl)amino]-3-methyle |
| 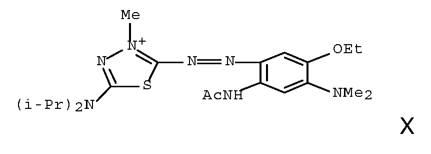 | 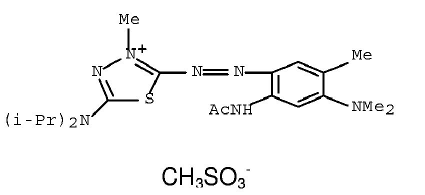 |
| Sel de 1,3,4-Thiadiazolium, 2-[[2-(acetylamino)-4-(dimethylamino)-5-ethoxyphenyl]azo]-5-[bis(1-methylethyl)amino]-3-methyle | Méthylsulfate de 1,3,4-Thiadiazolium, 2-[[2-(acetylamino)-4-(dimethylamino)-5-methylphenyl]azo]-5-[bis(1-methylethyl)amino]-3-methyle |
| 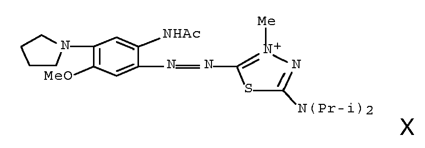 | 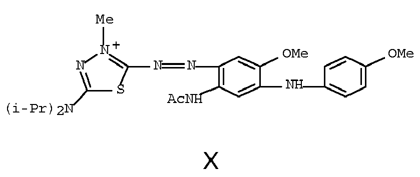 |
| Sel de 1,3,4-Thiadiazolium, 2-[[2-(acetylamino)-5-methoxy-4-(1-pyrrolidinyl)phenyl]azo]-5-[bis(1-methylethyl)amino]-3-methyle | Sel de 1,3,4-Thiadiazolium, 2-[[2-(acetylamino)-5-methoxy-4-[(4-methoxyphenyl)amino]phenyl]azo]-5-[bis(1-methylethyl)amino]-3-methyle |
| 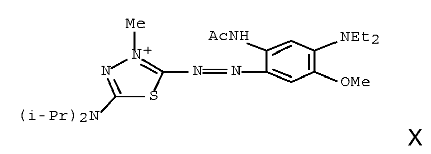 | 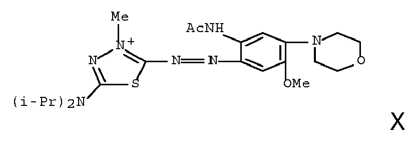 |
| Sel de 1,3,4-Thiadiazolium, 2-[[2-(acetylamino)-4-(diethylamino)-5-methoxyphenyl]azo]-5-[bis(1-methylethyl)amino]-3-methyle | Sel de 1,3,4-Thiadiazolium, 2-[[2-(acetylamino)-5-methoxy-4-(4-morpholinyl)phenyl]azo]-5-[bis(1-methylethyl)amino]-3-methyle |
| 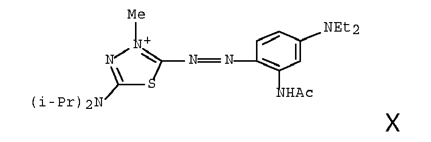 | 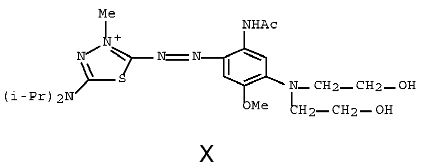 |
| Sel de 1,3,4-Thiadiazolium, 2-[[2-(acetylamino)-4-(diethylamino)phenyl]azo]-5- [bis(1-methylethyl)amino]-3-methyle | Sel de 1,3,4-Thiadiazolium, 2-[[2-(acetylamino)-4-[bis(2-hydroxyethyl)amino]-5-methoxyphenyl]azo]-5-[bis(1-methylethyl)amino]-3-methyle |
| 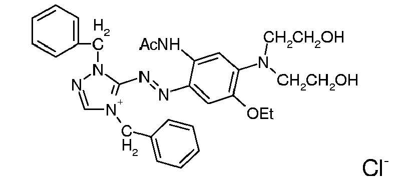 | 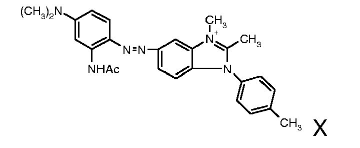 |
| Chlorure de 1 H-1,2,4-Triazolium, 5-[[2-(acetylamino)-4-[bis(2-hydroxyethyl)amino]-5-ethoxyphenyl]azo]-1,4-bis(phenylmethyle) | Sel de 1 H-Benzimidazolium, 5-[[2-(acetylamino)-4-(dimethylamino)phenyl]azo]-2,3-dimethyl-1-(4-methylphenyle) |
| 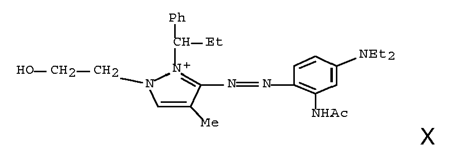 | 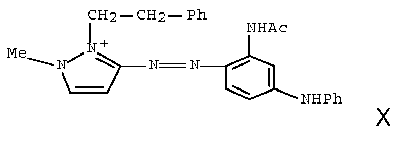 |
| Sel de 1 H-Pyrazolium, 3-[[2-(acetylamino)-4-(diethylamino)phenyl]azo]-1-(2-hydroxyethyl)-4-methyl-2-(1-phenylpropyle) | Sel de 1 H-Pyrazolium, 3-[[2-(acetylamino)-4-(phenylamino)phenyl]azo]-1-methyl-2- (2-phenylethyle) |
| 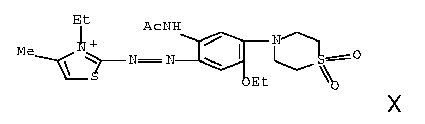 | 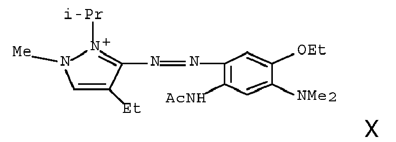 |
| Sel de Thiazolium, 2-[[2-(acetylamino)-4-(1,1-dioxido-4-thiomorpholinyl)-5-ethoxyphenyl]azo]-3-ethyl-4-methyle | Sel de 1 H-Pyrazolium, 3-[[2-(acetylamino)-4-(dimethylamino)-5-ethoxyphenyl]azo]-4-ethyl-1-methyl-2-(1-methylethyle) |
| 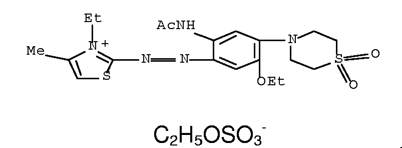 | 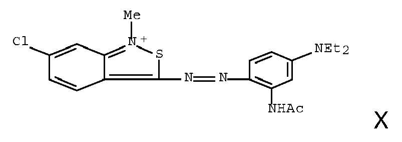 |
| Ethylsulfate de Thiazolium, 2-[(2- | Sel de 2,1 -Benzisothiazolium, 3-[[2- |
| acetamido-5-ethoxy-4-thiomorpholinophenyl)azo]-3-ethyl-4-methyl-S,S-dioxide | (acetylamino)-4-(diethylamino)phenyl]azo]-6-chloro-1-methyle |
| 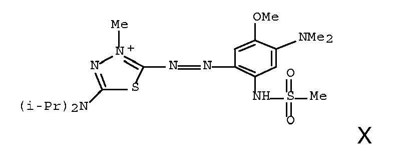 | 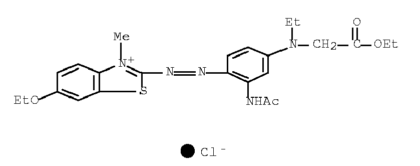 |
| Sel de 1,3,4-Thiadiazolium, 5-[bis(1-methylethyl)amino]-2-[[4-(dimethylamino)-5-methoxy-2-[(methylsulfonyl)amino]phenyl]azo]-3-methyle | Chlorure de Benzothiazolium, 2-[[2-(acetylamino)-4-[(2-ethoxy-2-oxoethyl)ethylamino]phenyl]azo]-6-ethoxy-3-methyle |
| 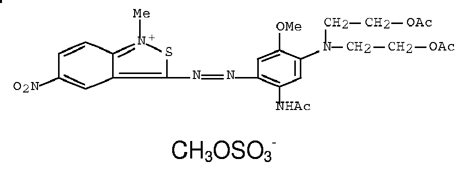 | 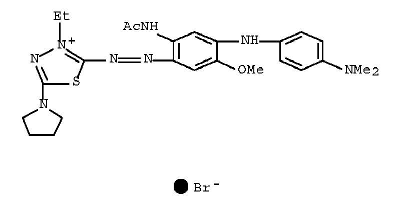 |
| Méthylsulfate de 2,1-Benzisothiazolium, 3-[[2-(acetylamino)-4-[bis[2-(acetyloxy)ethyl]amino]-5-methoxyphenyl]azo]-1-methyl-5-nitro | Bromure de 1 ,3,4-Thiadiazolium, 2-[[2-(acetylamino)-4-[[4-(dimethylamino)phenyl]amino]-5-methoxyphenyl]azo]-3-ethyl-5-(1-pyrrolidinyle) |
| 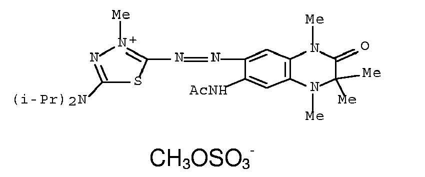 | 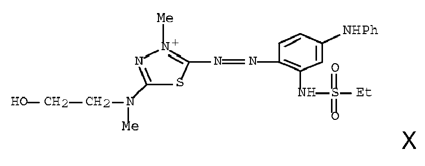 |
| Méthylsulfate de 1,3,4-Thiadiazolium, 2-[[7-(acetylamino)-1,2,3,4-tetrahydro-1,2,2,4-tetramethyl-3-oxo-6-quinoxalinyl]azo]-5-[bis(1-methylethyl)amino]-3-methyle | Sel de 1,3,4-Thiadiazolium, 2-[[2-[(ethylsulfonyl)amino]-4-(phenylamino)phenyl]azo]-5-[(2-hydroxyethyl)methylamino]-3-methyle |
| 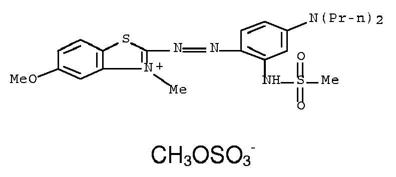 | 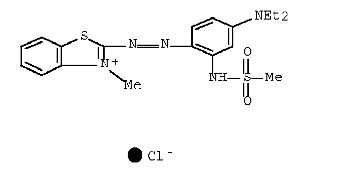 |
| Methylsulfate de Benzothiazolium, 2-[[4-(dipropylamino)-2-[(methylsulfonyl)amino]phenyl]azo]-5-methoxy-3-methyle | Chlorure de Benzothiazolium, 2-[[4-(diethylamino)-2-[(methylsulfonyl)amino]phenyl]azo]- 3-methyle |
| 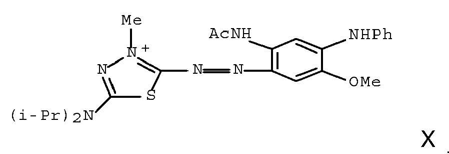 | 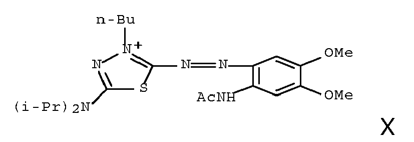 |
| Sel de 1,3,4-Thiadiazolium, 2-[[2-(acetylamino)-5-methoxy-4-(phenylamino)phenyl]azo]-5-[bis(1-methylethyl)amino]-3-methyle | Sel de 1,3,4-Thiadiazolium, 2-[[2-(acetylamino)-4,5-dimethoxyphenyl]azo]-5-[bis(1-methylethyl)amino]-3-butyle |
| 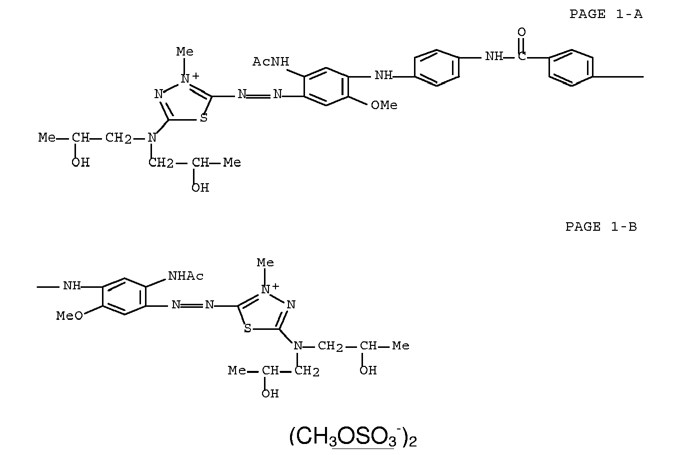 | |
| Bis(méthylsulfate)de de 1,3,4-Thiadiazolium, 2-[[2-(acetylamino)-4-[[4-[[[4-[[5-(acetylamino)-4- [[5-[bis(2-hydroxypropyl)amino]-3-methyl-1,3,4-thiadiazolium-2-yl]azo]-2-methoxyphenyl]amino]phenyl]amino] carbonyl]phenyl]amino]-5-methoxyphenyl]azo]-5-[bis(2-hydroxypropyl)amino]-3-methyle | |
| 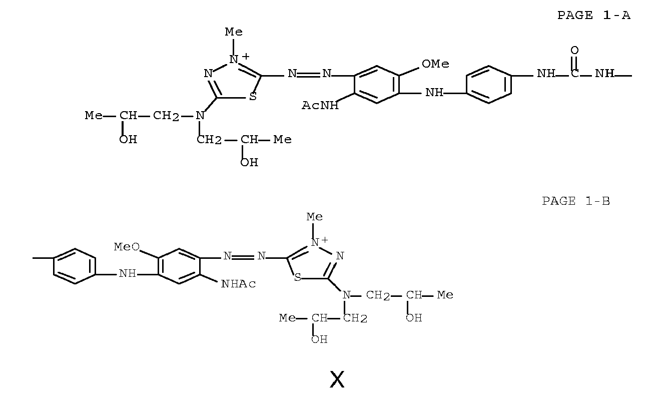 | |
| Sel de 1,3,4-Thiadiazolium, 2,2'-[carbonylbis[imino-4,1-phenyleneimino[2-(acetylamino)-5-methoxy-4,1-phenylene]azo]]bis[5-[bis(2-hydroxypropyl)amino]-3-methyle | |
| 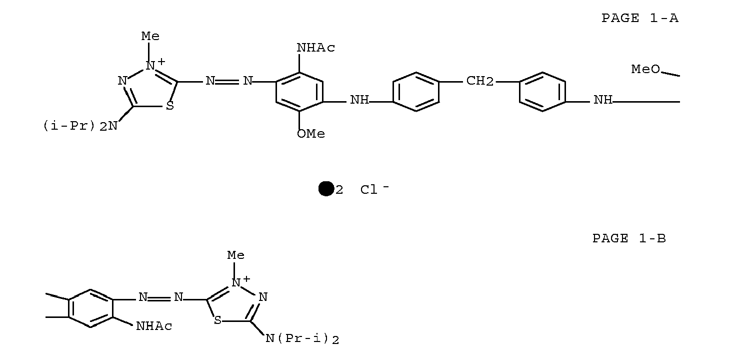 | |
| Dichlorure de 1,3,4-Thiadiazolium, 2,2'-[methylenebis[4,1-phenyleneimino[2-(acetylamino)- 5-methoxy-4,1-phenylene]azo]]bis[5-[bis(1-methylethyl)amino]-3-methyle | |
| 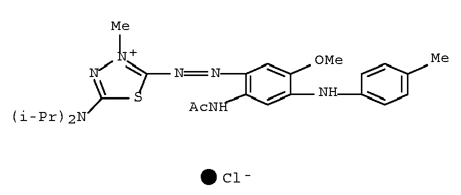 | 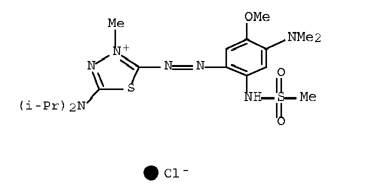 |
| Chlorure de 1,3,4-Thiadiazolium, 2-[[2-(acetylamino)-5-methoxy-4-[(4-methylphenyl)amino]phenyl]azo]-5-[bis(1-methylethyl)amino]-3-methyle | Chlorure de 1,3,4-Thiadiazolium, 5-[bis(1-methylethyl)amino]-2-[[4-(dimethylamino)-5-methoxy-2-[(methylsulfonyl)amino]phenyl]azo]-3-methyle |
| 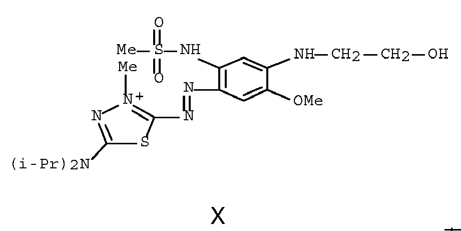 | 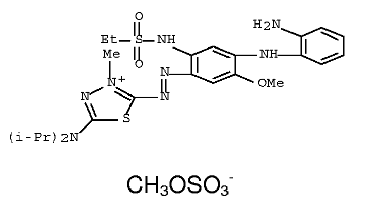 |
| Sel de 1,3,4-Thiadiazolium, 5-[bis(1-methylethyl)amino]-2-[[4-[(2-hydroxyethyl)amino]-5-methoxy-2-[(methylsulfonyl)amino]phenyl]azo]-3-methyle | Méthyl sulfate de 1,3,4-Thiadiazolium, 2-[[4-[(2-aminophenyl)amino]-2-[(ethylsulfonyl)amino]- 5-methoxyphenyl]azo]-5-[bis(1-methylethyl)amino]-3-methyle |
| 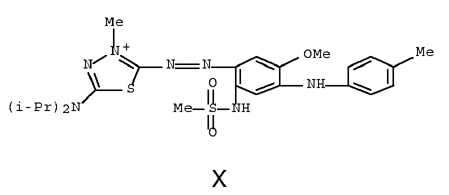 | 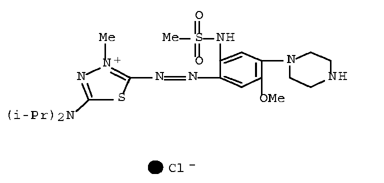 |
| Sel de 1,3,4-Thiadiazolium, 5-[bis(1-methylethyl)amino]-2-[[5-methoxy-4-[(4-methylphenyl)amino]-2-[(methylsulfonyl)amino]phenyl]azo]-3-methyle | Chlorure de 1,3,4-Thiadiazolium, 5-[bis(1-methylethyl)amino]-2-[[5-methoxy-2-[(methylsulfonyl)amino]-4-(1-piperazinyl)phenyl]azo]-3-methyle |
| 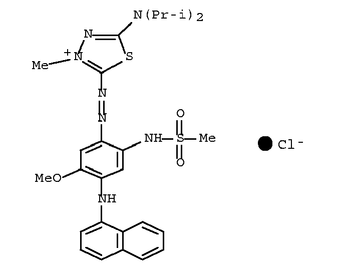 | 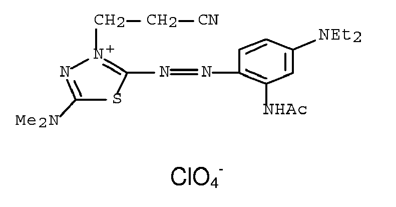 |
| Chlorure de 1,3,4-Thiadiazolium, 5-[bis(1-methylethyl)amino]-2-[[5-methoxy-2-[(methylsulfonyl)amino]-4-(1-naphthalenylamino)phenyl]azo]-3-methyle | Perchlorate de 1,3,4-Thiadiazolium, 2-[[2-(acetylamino)-4-(diethylamino)phenyl]azo]-3-(2-cyanoethyl)-5-(dimethylamino) |
| 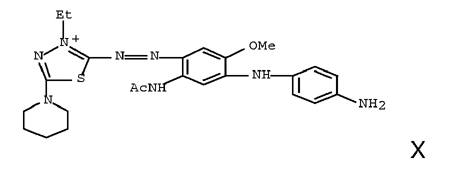 | 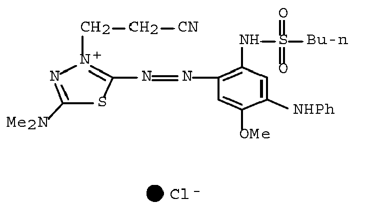 |
| Sel de 1,3,4-Thiadiazolium, 2-[[2-(acetylamino)-4-[(4-aminophenyl)amino]-5-methoxyphenyl]azo]-3-ethyl-5-(1-piperidinyl)-, ethyle | Chlorure de 1,3,4-Thiadiazolium, 2-[[2-[(butylsulfonyl)amino]-5-methoxy-4-(phenylamino)phenyl]azo]-3-(2-cyanoethyl)-5-(dimethylamino) |
| 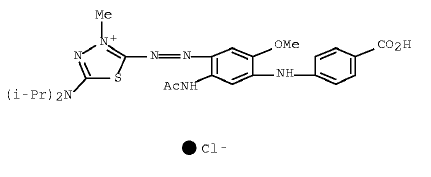 | 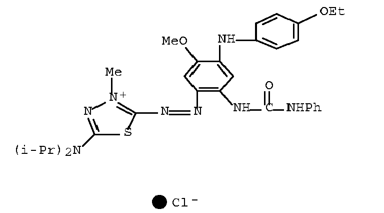 |
| Chlorure de 1,3,4-Thiadiazolium, 2-[[2-(acetylamino)-4-[(4-carboxyphenyl)amino]-5-methoxyphenyl]azo]-5-[bis(1-methylethyl)amino]-3-methyle | Chlorure de 1,3,4-Thiadiazolium, 5-[bis(1-methylethyl)amino]-2-[[4-[(4-ethoxyphenyl)amino]-5-methoxy-2-[[(phenylamino)carbonyl]amino]phenyl]azo ]- 3-methyle |
| 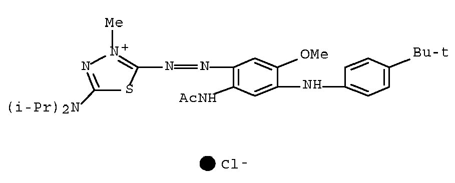 | 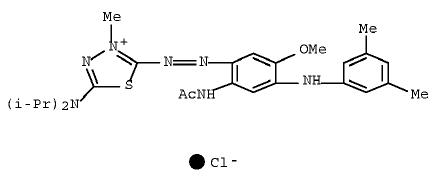 |
| Chlorure de 1,3,4-Thiadiazolium, 2-[[2-(acetylamino)-4-[[4-(1,1-dimethylethyl)phenyl]amino]-5-methoxyphenyl]azo]-5-[bis(1-methylethyl)amino]-3-methyle | Chlorure de 1,3,4-Thiadiazolium, 2-[[2-(acetylamino)-4-[(3,5-dimethylphenyl)amino]-5-methoxyphenyl]azo]-5-[bis(1-methylethyl)amino]-3-methyle |
| 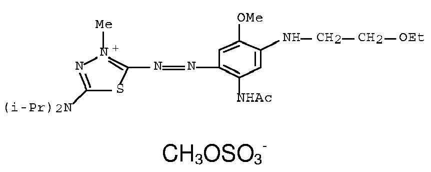 | 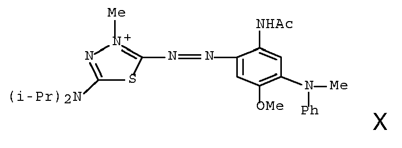 |
| methyl sulfate de 1,3,4-Thiadiazolium, 2-[[2-(acetylamino)-4-[(2-ethoxyethyl)amino]-5-methoxyphenyl]azo]-5-[bis(1-methylethyl)amino]-3-methyle | Sel de 1,3,4-Thiadiazolium, 2-[[2-(acetylamino)-5-methoxy-4-(methylphenylamino)phenyl]azo]-5-[bis(1-methylethyl)amino]-3-methyle |
| 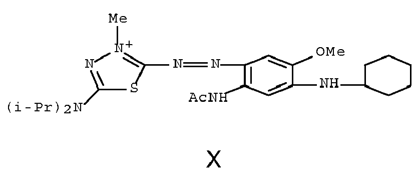 | 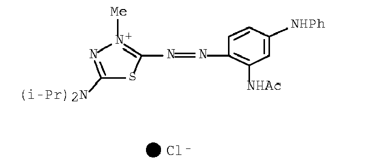 |
| Sel de 1,3,4-Thiadiazolium, 2-[[2-(acetylamino)-4-(cyclohexylamino)-5-methoxyphenyl]azo]-5-[bis(1-methylethyl)amino]-3-methyle | Chlorure de 1,3,4-Thiadiazolium, 2-[[2-(acetylamino)-4-(phenylamino)phenyl]azo]-5- [bis(1-methylethyl)amino]-3-methyle |
| 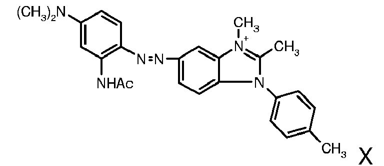 | 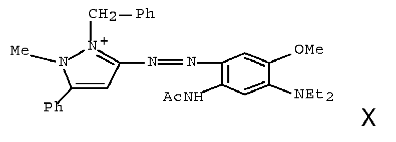 |
| Sel de 1H-Benzimidazolium, 5-[[2-(acetylamino)-4-(dimethylamino)phenyl]azo]-2,3-dimethyl-1-phenyle | Sel de 1H-Pyrazolium, 3-[[2-(acetylamino)-4-(diethylamino)-5-methoxyphenyl]azo]-1-methyl-5-phenyl-2-(phenylmethyle) |
| 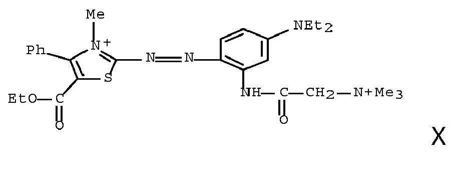 | 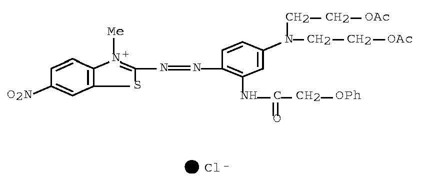 |
| Sel de Thiazolium, 2-[[4-(diethylamino)-2-[[(trimethylammonio)acetyl]amino]phenyl]azo]-5-(ethoxycarbonyl)-3-methyl-4-phenyle | Chlorure de Benzothiazolium, 2-[[4-[bis(2-hydroxyethyl)amino]-2-(2-phenoxyacetamido)phenyl]azo]-3-methyl-6-nitro |
| 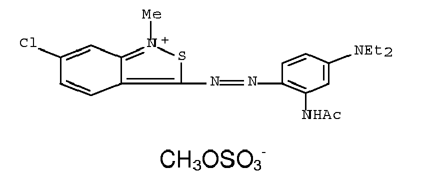 | 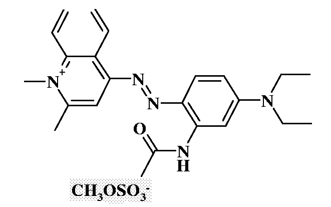 |
| methyl sulfate de 2,1-Benzisothiazolium, 3-[[2-acetamido-4-(diethylamino)phenyl]azo]-6-chloro-1-methyle | Méthosulfate de 4-(2-Acetylamino-4-diethylamino-phenylazo)-1,2-dimethyl-quinolinium |

A noter que la nature des contre-ions présents dans le tableau ci-dessus est simplement donnée à titre indicatif ; celui-ci n'étant pas critique.

Selon un mode de réalisation particulier de l'invention, la composition tinctoriale comprenant le composé de formule (I) présente une teneur en ce composé variant entre 0,001 et 10% en poids par rapport au poids de la composition tinctoriale, et de préférence entre environ 0,05 et 5% en poids par rapport au poids de la composition tinctoriale.

L'invention a de même pour objet un procédé de coloration qui comprend la mise en contact d'une composition tinctoriale comprenant au moins un composé de formule (I) précédemment défini, sur les fibres kératiniques humaines, sèches ou humides.

La composition tinctoriale mise en oeuvre dans le procédé selon l'invention peut éventuellement comprendre au moins un colorant direct additionnel différents des composés de formule (I). Celui-ci peut être choisi de préférence parmi les espèces cationiques ou non ioniques.

A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.

Parmi les colorants directs azoïques utilisables selon l'invention on peut citer les colorants azoïques cationiques décrits dans les demandes de brevets WO 95/15144, WO 95/01772, EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

S'ils sont présents, la teneur en colorant(s) direct(s) additionnels dans la composition tinctoriale varie en général de 0,001 à 20% en poids par rapport au poids de la composition, et de préférence de 0,01 à 10% en poids par rapport au poids de la composition.

La composition tinctoriale mise en oeuvre peut de plus comprendre une ou plusieurs bases d'oxydation.

Ces bases d'oxydation peuvent être choisies parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques et les dérivés de type pyrazolo[1,2a]pyrazol-1-one.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés de type pyrazolo[1,2a]pyrazol-1-one, on peut citer les composés comme le 2,3-diamino-6,7-dihydro,1H-5H-pyrazolo[1,2a]pyrazol-1-one.

La ou les bases d'oxydation sont en général présentes en quantité comprise entre 0,001 à 10 % en poids par rapport au poids de la composition tinctoriale, et plus préférentiellement de 0,005 à 6 % en poids par rapport au poids de la composition tinctoriale.

La composition tinctoriale mise en oeuvre peut aussi comprendre un ou plusieurs coupleurs conventionnellement utilisés pour la teinture de fibres kératiniques humaines.

Parmi ces coupleurs, on peut notamment citer les métaphénylènediamines, les méta-aminophénols, les métadiphénols, les coupleurs naphtaléniques et les coupleurs hétérocycliques.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.

Dans ladite composition, le ou les coupleurs, s'ils sont présents, représentent en général une quantité comprise entre 0,001 et 10 % en poids par rapport au poids de la composition tinctoriale et plus préférentiellement de 0,005 à 6 % en poids par rapport au poids de la composition tinctoriale.

D'une manière générale, les sels d'addition avec un acide utilisables pour les bases d'oxydation et les coupleurs sont notamment choisis parmi les chlorhydrates, les bromhydrates, les sulfates, les citrates, les succinates, les tartrates, les lactates, les tosylates, les benzènesulfonates, les phosphates et les acétates.

La composition tinctoriale mise en oeuvre comprend un milieu approprié pour la teinture, appelé aussi support de teinture. Il est généralement constitué par de l'eau ou par un mélange d'eau et d'au moins un solvant organique pour solubiliser les composés qui ne seraient pas suffisamment solubles dans l'eau.

Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants usuels décrits ci-dessus, s'ils sont présents, représentent habituellement de 1 à 40 % en poids, plus préférentiellement de 5 à 30 % en poids, par rapport au poids total de la composition tinctoriale.

La composition tinctoriale peut également renfermer divers adjuvants utilisés classiquement dans les compositions pour la teinture des cheveux, tels que des agents tensioactifs comme les tensioactifs anioniques, cationiques, non ioniques, amphotères, zwittérioniques ou leurs mélanges ; des polymères anioniques, cationiques, non ioniques, amphotères, zwittérioniques ou leurs mélanges ; des agents épaississants minéraux ; des polymères épaississants, et en particulier les polymères épaississants associatifs anioniques, cationiques, non ioniques et amphotères ; des agents antioxydants ; des agents de pénétration ; des agents séquestrants ; des parfums ; des tampons ; des agents dispersants ; des agents de conditionnement tels que par exemple des silicones volatiles ou non volatiles, modifiées ou non modifiées ; des agents filmogènes ; des céramides ; des agents conservateurs ; des agents opacifiants, etc.

Ces adjuvants ci dessus sont en général présents en quantité comprise pour chacun d'eux entre 0,01 et 20 % en poids par rapport au poids de la composition tinctoriale.

Le pH de la composition tinctoriale est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 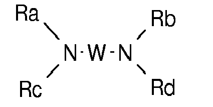 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques humaines.

La composition mise en oeuvre dans le procédé selon l'invention peut en outre comprendre au moins un agent oxydant. On parle dans ce cas de composition prête à l'emploi.

Par composition prête à l'emploi, on entend au sens de la présente invention, la composition destinée à être appliquée immédiatement sur les fibres kératiniques, c'est-à-dire qu'elle peut être stockée telle quelle avant utilisation ou résulter du mélange extemporané de deux ou plusieurs compositions.

Habituellement, ladite composition prête à l'emploi est obtenue en mélangeant la composition tinctoriale exempte d'agent oxydant avec une composition oxydante.

A noter qu'il ne serait pas exclu d'appliquer simultanément ou successivement une composition comprenant au moins un composé de formule (I) exempte d'agent oxydant, et une composition comprenant au moins un agent oxydant.

L'agent oxydant peut être n'importe quel agent de ce type utilisé de façon classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons telles que les uricases et les oxygénases à 4 électrons comme les laccases. L'utilisation du peroxyde d'hydrogène est particulièrement préférée.

La teneur en agent oxydant est en général comprise entre 1 et 40 % en poids, par rapport au poids de la composition prête à l'emploi de préférence entre 1 et 20 % en poids par rapport au poids de la composition prête à l'emploi.

La composition oxydante peut également renfermer divers adjuvants utilisés classiquement dans les compositions pour la teinture des fibres kératiniques humaines et tels que définis précédemment.

Généralement, la composition oxydante utilisée est une composition aqueuse et peut se trouver sous la forme d'une solution ou encore d'une émulsion.

Habituellement, on mélange la composition tinctoriale exempte d'agent oxydant avec environ 0,5 à 10 équivalents en poids de la composition oxydante.

Notons que le pH de la composition prête à l'emploi est plus particulièrement compris entre 4 et 12, de préférence entre 7 et 11,5.

Le pH de la composition prête à l'emploi peut être réglé au moyen d'un agent alcalinisant ou acidifiant, notamment choisis parmi ceux mentionnés auparavant.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques humaines.

Selon un mode de réalisation particulier, la composition selon l'invention est exempte de base d'oxydation et de coupleur.

La composition mise en contact avec les fibres kératiniques humaines peut éventuellement comprendre au moins un agent oxydant.

La composition est donc appliquée sur les fibres kératiniques, sèches ou humides, puis laissée pendant un temps de pose suffisant pour obtenir la coloration recherchée.

Quelle que soit la variante retenue (avec ou sans agent oxydant) le temps de pose est en général compris entre quelques secondes et 30 minutes, de préférence entre 3 et 15 minutes.

La température à laquelle la composition est laissée agir, est en général comprise entre 15 et 220°C, plus particulièrement entre 15 et 80°C, de préférence entre 15 et 40 °C.

A l'issue du temps de pose, la composition est éliminée par un rinçage à l'eau, suivi d'un lavage avec un shampooing, puis éventuellement d'un séchage.

L'exemple qui suit sert à illustrer l'invention sans toutefois présenter un caractère limitatif.

### EXEMPLES

On prépare les trois compositions suivantes :

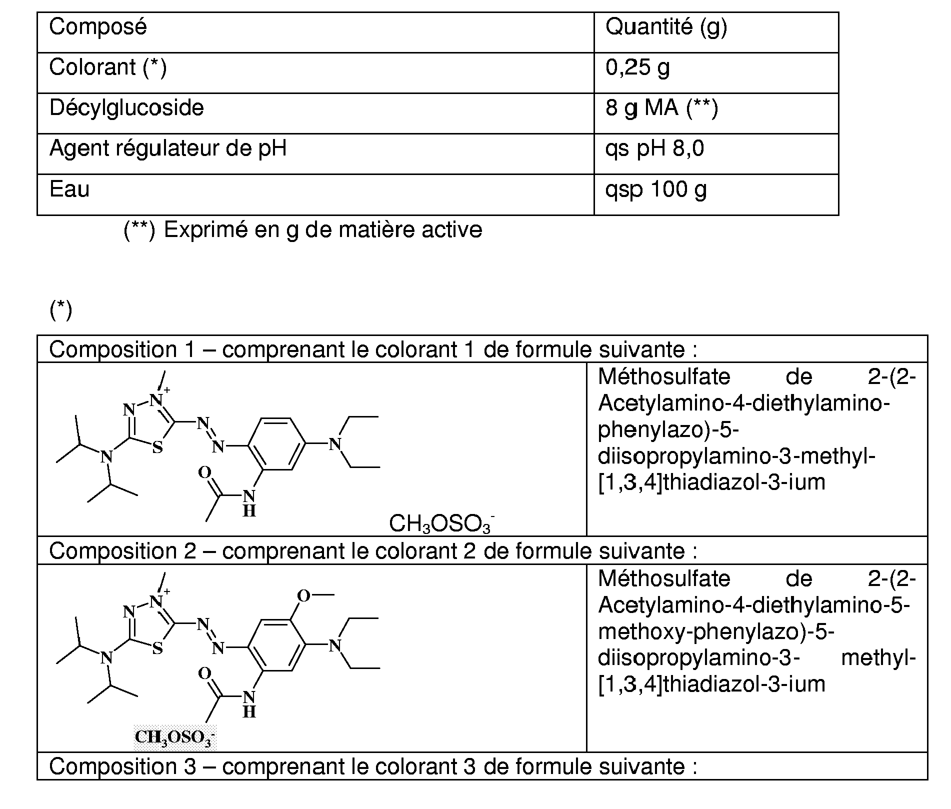

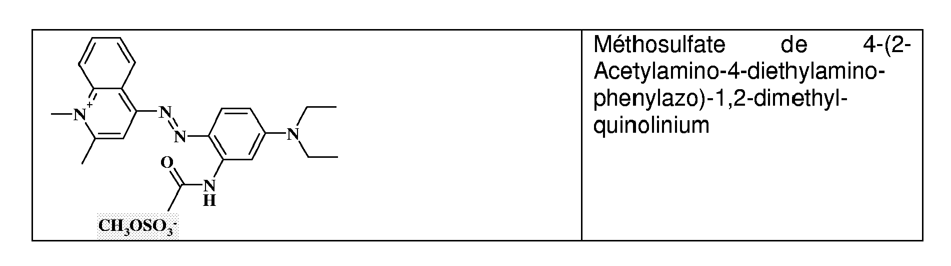

Chacune de ces trois compositions est appliquée sur des mèches de cheveux naturels à 90 % de blancs, à température ambiante pendant 30 minutes.

A l'issue du temps de pause, chaque mèche est rincée et lavée avec un shampoing, rincée à nouveau puis séchée.

On obtient les couleurs suivantes :
- Colorant 1: bleu rougeâtre
- Colorant 2: bleu
- Colorant 3: bleu

## Revendications

1. Utilisation pour la coloration de fibres kératiniques humaines, d'une composition tinctoriale comprenant au moins un composé de formule (I) suivante, ou l'une de ses formes mésomères : 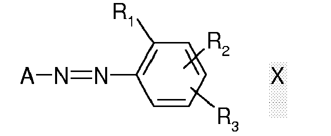 formule dans laquelle :
* **A** représente un hétérocycle aromatique monocationique éventuellement substitué comprenant 5 à 6 chaînons, comprenant au moins un atome d'azote et au moins un autre hétéroatome choisi parmi l'azote, le soufre ; ledit hétérocycle étant éventuellement condensé avec un noyau aromatique non hétérocyclique à 6 chaînons éventuellement substitué ; ou représente un hétérocycle aromatique monocationique éventuellement substitué, à 6 chaînons, comprenant un atome d'azote, condensé avec un noyau aromatique non hétérocyclique à 6 chaînons éventuellement substitué ; le groupement A pouvant être rattaché à la fonction azoïque par l'intermédiaire de l'un des chaînons de l'hétérocycle ou du noyau aromatique non hétérocyclique ;
* **R₁** représente :
- -NH-CO-R'₁ avec R'₁ représentant :
• un radical alkyle en C₁-C₆, linéaire ou ramifié, éventuellement porteur d'un groupement trialkyle (C₁-C₄) ammonium ou éventuellement porteur un groupement phénoxy ;
• un groupement amino éventuellement porteur d'un ou plusieurs groupements identiques ou différents, choisi parmi les groupements alkyle en C₁-C₄, hydroxyalkyle en C₁-C₄, phényle ;
- -NH-SO₂-R'₂ avec R'₂ représentant un radical alkyle en C₁-C₆, linéaire ou ramifié ;
* **R₂** représente :
- un atome d'hydrogène,
- un groupement alcoxy en C₁-C₆ ;
- un groupement amino portant un ou deux substituants identiques ou différents, choisis parmi :
• les radicaux alkyle en C₁-C₆, linéaires ou ramifiés, éventuellement substitués par un groupement hydroxyle ; par un groupement alcoxy en C₁-C₄ ; par un groupement alcoxy(C₁-C₄)carbonyle ; par un groupement acyl(C₂-C₄)oxy ; par un groupement phényle ; par un groupement cyano ;
• les deux radicaux alkyle dudit groupement amino pouvant former avec l'atome d'azote auquel ils sont rattachés, un hétérocycle à 5 ou 6 chaînons, saturé ou insaturé, comprenant éventuellement un autre hétéroatome identique ou différent de l'azote, comme par exemple l'oxygène ou encore un atome de soufre porteur de deux atomes d'oxygène et éventuellement substitué par un ou plusieurs radicaux alkyle en C₁-C₄ ;
• les radicaux alkyle cycliques en C₅-C₇ ;
• les radicaux naphtyle ;
• un hétérocycle azoté à 5 ou 6 chaînons, éventuellement insaturé, rattaché au noyau aromatique par l'intermédiaire d'un atome d'azote, comprenant éventuellement un autre hétéroatome choisi parmi l'azote ou l'oxygène, ledit hétérocycle étant éventuellement substitué par un ou plusieurs radicaux alkyle en C₁-C₄ linéaires ou ramifiés ;
• les radicaux phényle éventuellement substitué par un ou plusieurs groupements alcoxy en C₁-C₄, hydroxyalcoxy en C₁-C₄, cyano, alkyle en C₁-C₆ linéaire, ramifié ou cyclique, hydroxyle, amino, amino substitué par un ou deux radicaux alkyle en C₁-C₄ identiques ou non, alkyl(C₁-C₄)carbonyle, acétylamino, aminocarbonyle, carboxy, les atomes d'halogène ;
- un groupement amino monosubstitué par un radical phényle éventuellement porteur d'un groupement alcoxy en C₁-C₄ linéaire ou ramifié, substitué par un groupement -D-NH-Φ(R₁)(R₃)-N=N-A avec D représentant un groupement -alkylène(C₁-C₂)-Φ- ou -NHCO-Φ- ou -O-alkylène (C₁-C₂)-O-Φ- ou -NH-CO-NH-Φ-ou une simple liaison ; R₁, et les hétérocycles A ayant la même définition que précédemment et choisis de préférence de telle sorte que les radicaux R₁ et les hétérocycles A soient respectivement identiques et placés dans les mêmes positions dans la molécule ; Φ représentant un radical phényle ; R₃ étant défini ci-dessous ;
* **R₃** représente :
- un atome d'hydrogène ;
- un radical alkyle en C₁-C₆ linéaire ou ramifié ;
- un groupement alcoxy en C₁-C₆ ;
- un groupement amino éventuellement porteur d'un ou plusieurs radicaux alkyle en C₁-C₄, hydroxyalkyle en C₁-C₄, phényle, identiques ou non ;
* **lorsque R₂ et R₃** représentent deux groupements amino disubstitués par deux radicaux alkyle identiques ou non, portés par deux atomes de carbone adjacents du noyau aromatique, alors lesdits radicaux R₂ et R₃ peuvent former avec les atomes de carbone auquel chacun est rattaché, un hétérocycle à 6 chaînons saturé éventuellement substitué par un ou deux radicaux alkyle en C₁-C₄, linéaires ou ramifiés, identiques ou non ; l'un des atomes de carbone dudit hétérocycle pouvant être remplacé par un groupement carbonyle ;
* au moins l'un des deux radicaux R₂ ou R₃ étant différent de l'hydrogène ;
* **X** représente un anion ou un mélange d'anions cosmétiquement acceptables.

2. Utilisation selon la revendication précédente, **caractérisée en ce que** le composé de formule (I) est tel que R₃ se trouve en position méta par rapport à la fonction azoïque.

3. Utilisation selon l'une quelconque des revendications 1 à 3, **caractérisée en ce que** le composé de formule (I) est tel que R₂ se trouve en position para par rapport à la fonction azoïque.

4. Utilisation selon la revendication précédente, **caractérisée en ce que** le composé de formule (I) est tel que **A** représente un hétérocycle choisi parmi les groupements imidazolium, pyrimidinium, pyrazolium, triazolium, thiazolium, thiadiazolium, quinolinium, benzimidazolium, benzoisothiazolium, benzothiazolium, éventuellement substitués.

5. Utilisation selon la revendication précédente, **caractérisée en ce que** le composé de formule (I) est tel que **A** représente un hétérocycle choisi parmi les groupements thiadiazolium, pyrazolium, thiazolium, benzimidazolium, benzoisothiazolium, benzothiazolium, triazolium, éventuellement substitués.

6. Utilisation selon l'une quelconque des revendications précédentes, **caractérisée en ce que** le composé de formule (I) est tel que **A** représente l'un des hétérocycles suivants :
| | |
|---|---|
| 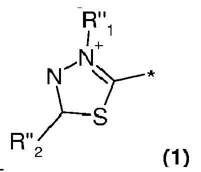 | 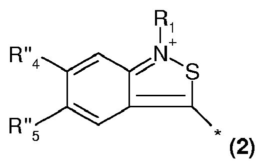 |
| 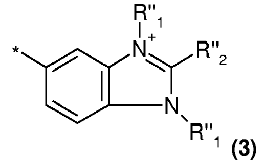 | 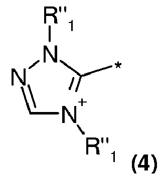 |
| 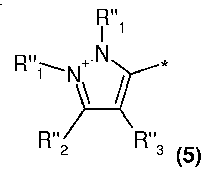 | 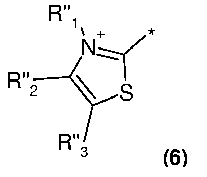 |
| 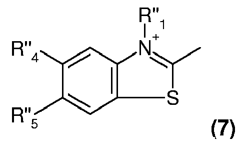 | |
Dans lesquels
R"₁ représente :
- un radical alkyle en C₁-C₆ éventuellement substitué par un groupement hydroxyle ; par un groupement cyano, par un groupement aminocarbonyle ;
R"₂, R"₃, identiques ou différents, représentent :
- un atome d'hydrogène ;
- un groupement alkyle en C₁-C₆ linéaire ou ramifié ;
- un groupement phényle ;
- un groupement amino éventuellement porteur d'un ou deux radicaux identiques ou différents, choisis parmi :
• les radicaux alkyle linéaires ou ramifiés en C₁-C₆ éventuellement substitués par un groupement hydroxyle, cyano ; ou cycliques en C₅-C₇ ;
• les radicaux phényle ;
• les groupements tétrahydro-1,1-dioxydo-3-thiényle ;
• les deux radicaux alkyle du groupement amino pouvant former avec l'atome d'azote auquel ils sont rattachés, un hétérocycle de 5 à 7 chaînons comprenant éventuellement un autre hétéroatome différent ou non de l'azote ;
- un groupement alcoxy(C₁-C₆)carbonyle ;
R"₄, R"₅, identiques ou différents, représentent :
- un atome d'hydrogène ;
- un groupement alkyle en C₁-C₆ linéaire ou ramifié ;
- un groupement alcoxy en C₁-C₆ linéaire ou ramifié ;
- un atome d'halogène, de préférence le chlore ;
- un groupement nitro.

7. Utilisation selon l'une quelconque des revendications précédentes, **caractérisés en ce que** le composé de formule (I) est tel que X est choisi parmi les halogénures tels que chlorures, bromures, fluorures, iodures ; les hydroxyde ; les sulfates ; les hydrogénosulfates ; les alkyl(C₁-C₆)sulfates ; les phosphates ; les carbonates ; les hydrogénocarbonates ; les perchlorates ; les acétates ; les tartrates ; les citrates, les oxalates ; les alkyl(C₁-C₆)sulfonates tels que le méthylsulfonate ; les arylsulfonates substitués ou non par un radical alkyle en C₁-C₄ tels que par exemple un 4-toluylsulfonate.

8. Utilisation selon l'une quelconque des revendications précédentes, **caractérisés en ce que** la teneur en composé de formule (I) représente de 0,001 à 10% en poids par rapport au poids de la composition, de préférence de 0,05 à 5% en poids par rapport au poids de la composition.

9. Procédé de coloration de fibres kératiniques consistant à mettre en contact une composition comprenant au moins un composé de formule (I) tel que défini à l'une quelconque des revendications précédentes, avec lesdites fibres, sèches ou humides, pendant une durée suffisante pour obtenir l'effet souhaité.

10. Procédé selon la revendication précédente, **caractérisé en ce que** la composition comprend au moins un colorant direct additionnel différent du composé de formule (I), au moins une base d'oxydation éventuellement associée à au moins un coupleur.

11. Procédé selon la revendication précédente, **caractérisé en ce que** le colorant direct additionnel est un colorant cationique ou non ionique, choisi parmi les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

12. Procédé selon l'une quelconque des revendications 9 à 13, **caractérisée en ce que** la composition comprend au moins un agent oxydant.

13. Composition tinctoriale comprenant, dans un milieu approprié pour la teinture des fibres kératiniques humaines, au moins un colorant de formule (I) tel que défini dans l'une des revendications 1 à 7, et au moins un adjuvant choisi parmi les agents tensioactifs ; les polymères anioniques, cationiques, non ioniques, amphotères, zwittérioniques ou leurs mélanges ; les agents épaississants minéraux ; les polymères épaississants ; les agents antioxydants ; les agents de pénétration ; les agents séquestrants ; les parfums ; les tampons ; les agents dispersants ; les agents de conditionnement ; les agents filmogènes ; les céramides ; les agents conservateurs ; les agents opacifiants.

14. Composition tinctoriale selon la revendication précédente, **caractérisée en ce qu'**elle comprend au moins un colorant direct différent du composé de formule (I) cationique ou non ionique.

15. Composition tinctoriale selon l'une des revendications 13 ou 14 **caractérisée en ce qu'**elle comprend au moins une base d'oxydation éventuellement associée à au moins un coupleur.
